# FASCICULE DE BREVET EUROPEEN

(11) **EP 2 234 670 B1**
(45) Date de publication et mention de la délivrance du brevet: **12.09.2012**
(21) Numéro de dépôt: 08868867.6
(22) Date de dépôt: 30.12.2008
(51) Int. Cl.: A61Q 3/02, A61K 8/37

(54) **COMPOSITION COSMÉTIQUE ANHYDRE**
WASSERFREIE KOSMETISCHE ZUSAMMENSETZUNG
ANHYDROUS COSMETIC COMPOSITION

(30) Priorité: 02.01.2008 EP 08150008
(43) Date de publication de la demande: 06.10.2010
(73) Titulaire: International Lacquers S.A., 3225 Bettembourg (LU)
(72) Inventeur: DEROSIER, Frédéric, F-57000 Metz (FR); DELAS, Christophe, L-3926 Mondercange (LU)
(74) Mandataire: Office Freylinger
(86) Numéro de dépôt international: PCT/EP2008/068368
(87) Numéro de publication internationale: WO 2009/083590

(56) Documents cités:
- EP-A- 1 477 154
- EP-A- 1 683 510
- EP-A- 1 690 523
- US-A- 5 882 636
- US-A1- 2001 007 676
- US-A1- 2006 018 854
- US-A1- 2007 258 933
- US-A1- 2007 258 934

## Description

### Domaine technique

La présente invention concerne une composition cosmétique anhydre applicable sur les ongles et qui forme un film dur, brillant et tenace.

### Etat de la technique

Les différentes propriétés que l'on demande à un vernis à ongles sont les suivantes :
- Bonne application, avec un pinceau de taille et forme adéquate. Le pinceau permet d'obtenir une surface lisse, sans stries avec une épaisseur constante afin d'obtenir un film et une coloration homogènes.
- Un temps de séchage approprié de l'ordre de quelques minutes.
- Une durabilité dans le temps que l'on définit par la tenue en évitant l'écaillement du vernis ou son usure en bout d'ongle.
- Une brillance importante à l'application, c'est à dire quelques minutes après séchage.
- Une tenue de la brillance dans le temps, c'est à dire une brillance diminuant le moins possible dans les jours qui suivent l'application.

Le paramètre d'application est plus particulièrement en relation avec des notions de viscosité et de rhéologie. La viscosité de la formulation dépend principalement des différents ingrédients utilisés dans la formulation et de leur dosage au sein de cette formulation. L'application peut également dépendre des matières colorantes utilisées dans la formulation colorée mais aussi de la qualité de l'applicateur.

Le temps de séchage est principalement dépendant des solvants utilisés dans la formulation et en particulier leur température d'ébullition et leur tension de vapeur. L'utilisation de différents solvants ayant des propriétés physico-chimiques différentes permet de moduler le temps de séchage et de durcissement du film qui peut également dépendre des ingrédients utilisés. D'autres ingrédients comme les résines et leur quantité utilisée dans la formulation peut influencer le temps de séchage.

La durabilité du vernis sur l'ongle ou tenue est principalement due aux agents non volatiles de la formulation tels que les résines, la nitrocellulose ou les différents additifs annexes.

La brillance est apportée par les différents constituants non volatiles du vernis à ongles comme les résines ou la nitrocellulose. Toutefois, ce paramètre est fortement affecté par les composés solides non solubles compris dans la formulation. Parmi ces composés solides non solubles, on citera notamment les pigments qui entraînent par leurs caractéristiques physico-chimiques une diminution de la brillance par rapport à la même formulation non pigmentée. Malgré les différents procédés engagés lors de la préparation de ces pigments, une diminution de la brillance est clairement notée. Les différentes étapes mises en oeuvre dans la préparation des pigments avant leur utilisation dans le vernis à ongles permettent plusieurs actions. Ces actions sur les pigments sont les suivantes :
- Une action de mouillage, qui permet de déplacer et d'éliminer l'air ou l'humidité qui se trouve à la surface du pigment.
- Une action de broyage qui permet de casser :
   ○ les agglomérats de pigments ; les agglomérats correspondent à l'association de structures primaires de pigments par des contacts de surface.
   ○ les agrégats de pigments ; les agrégats correspondent plutôt à une structure secondaire ayant pour origine l'association des structures primaires par des contacts bord à bord.
- Une action de dispersion, qui correspond plus particulièrement à la distribution homogène des particules primaires des pigments afin d'éviter la reformation des structures secondaires

A ces différentes actions sur le pigment lui-même, s'ajoute l'utilisation d'ingrédients annexes utilisés en faible quantité mais dont le pourcentage d'utilisation dépend directement de la nature (organique ou non) du pigment à formuler. Ces ingrédients appelés mouillants ou dispersants ont en général des structures bifonctionnelles. L'une des fonctions, ayant une forte affinité pour la surface du pigment, s'oriente vers celle-ci et se lie à elle par des liaisons chimiques ou des liaisons d'énergie plus faible de type Van der Waals. La seconde fonction s'oriente quant à elle vers la phase continue, composée généralement de solvants et de résines.

Ces mouillants assurent ainsi une meilleure affinité des pigments vers le milieu organique et évite également la reformation des structures secondaires décrites plus haut. La reformation de ces structures secondaires peut en effet se révéler problématique d'un point de vue colorimétrique mais peut aussi conduire à une instabilité de la formule caractérisée notamment par une sédimentation des pigments, les structures secondaires étant plus lourdes que les structures primaires.

Il est connu de l'homme de l'art une grande variété de mouillants/dispersants, que ce soit dans le milieu cosmétique ou dans le milieu de la peinture. Ces mouillants/dispersants peuvent se présenter sous la forme de composés simples ou encore sous la forme de composés polymériques ayant des chaînes latérales fonctionnalisées.

On notera notamment le brevet US 4,302,442 qui décrit l'utilisation de phosphatide, comme la lécithine, pour empêcher la migration des pigments, la lécithine est généralement utilisée en association avec un acide organique ou inorganique.

On citera également le brevet US 5,133,966 qui décrit l'utilisation d'acides gras et leurs sels dans la formulation pour améliorer la dispersion des pigments en association avec une résine dite « carrier resin ».

Même si ces inventions montrent que l'utilisation de différents dérivés permettent une amélioration accrue de la stabilité des pigments dans la formulation empêchant ainsi la réagglomération, les phénomènes de flottation ou les phénomènes de sédimentation, rien n'est mentionné concernant la brillance de la formulation colorée finale.

### Objet de l'invention

Un objet de la présente invention est par conséquent de proposer des formulations avec une brillance augmentée par rapport aux formulations connues.

Conformément à l'invention, cet objectif est atteint par une composition selon la revendication 1.

### Description générale de l'invention

Afin de résoudre le problème mentionné ci-dessus, la présente invention propose une composition cosmétique anhydre pour vernis à ongles, comprenant au moins un solvant organique cosmétiquement acceptable et une quantité efficace d'au moins un agent d'amélioration de la brillance, dans laquelle le au moins un agent d'amélioration de la brillance est un dérivé de pentaérythritol choisi parmi les tétraesters d'acides gras de pentaérythrityle et en ce que le au moins un agent d'amélioration de la brillance représente 0.01 à 1 % en poids de la composition totale.

En effet, les inventeurs ont découvert d'une manière surprenante que lesdits dérivés de pentaérythritol permettaient de réaliser l'objectif d'augmenter nettement la brillance de compositions cosmétiques anhydres après application.

Par « quantité efficace d'au moins un agent d'amélioration de la brillance », on entend une quantité suffisante pour obtenir une amélioration notable et significative de la brillance après application de la composition cosmétique. Cette quantité minimale en agent d'amélioration de la brillance à mettre en oeuvre, qui peut varier selon la nature du solvant cosmétiquement acceptable retenu pour la composition, ainsi qu'en fonction des autres ingrédients choisis, peut être déterminée sans aucune difficulté au moyen d'un test classique de mesure de la brillance, tel que celui donné dans les exemples ci-après. En pratique, le ou les agent(s) d'amélioration de la brillance représente(nt) 0.01 à 1 % en poids, de préférence 0.02 à 0.8 % en poids, en particulier de 0.05 à 0.5 % en poids de la composition totale.

Par conséquent, l'avantage de la présente invention consistant à proposer l'utilisation de dérivés d'acides gras de pentaérythrityle est l'accroissement significatif de la brillance de la formulation après application. Comme avantage supplémentaire, on doit noter que bien que la tenue de la brillance ne semble pas directement améliorée, une brillance plus importante au départ permet néanmoins de bénéficier d'une brillance accrue les jours suivant l'application.

D'autre part, l'ajout des agents d'amélioration de la brillance selon l'invention n'affecte pas négativement les autres qualités des compositions, à savoir les bonnes caractéristiques d'application, le temps de séchage et la durabilité dans le temps.

Un avantage additionnel important de la présente invention est que les compositions cosmétiques anhydre pour vernis à ongles, à brillance améliorée peuvent comprendre les ingrédients habituellement utilisés par l'homme de l'art. L'homme de métier n'est donc pas obligé de reconsidérer tous les ingrédients et leurs effets, s'il souhaite utiliser les présents agents d'améliorations de la brillance. Les présents agents d'amélioration de la brillance présentent par conséquent une très bonne compatibilité tant avec les solvants, qu'avec les ingrédients courants dans le domaine.

Les solvants utilisables dans l'invention sont choisis par exemple parmi les suivants : l'acétate de butyle, l'acétate d'isobutyle, l'acétate de propyle, l'acétate de méthyle, l'acétate d'éthyle, le toluène, l'isopropanol, le diacétone alcool, l'éthanol dénaturé (avec la méthyléthylcétone ou le diéthyléther), le n-butanol, les hydrocarbures linéaires ou cycliques, comme l'hexane, le cyclohexane, l'heptane, l'isododécane. On citera également les dérivés de paraffine de type Isopar qui sont des dérivés paraffiniques qui ont des chaînes carbonées de taille variable, ainsi que les glycols comme l'éthylène glycol, le propylène glycol, le dipropylène glycol ou le tripropylène glycol.

Les solvants ou leurs mélanges peuvent être utilisés entre 5 et 80 % en poids de la formulation totale. Ils sont sélectionnés selon leurs propriétés physico-chimiques (température d'ébullition par exemple) et leur aptitude à solubiliser les autres constituants de la formulation. Ils seront également sélectionnés selon leurs propriétés organoleptiques (odeur principalement) et leurs possibles utilisations selon les réglementations en vigueur.

Les compositions selon l'invention sont anhydres, c'est-à-dire qu'elles ne contiennent a priori pas d'eau. Il est néanmoins clair que de l'eau peut se trouver en de faibles quantités dans les compositions finales, notamment introduites via l'incorporation de certains ingrédients. Par conséquent, le terme « anhydre » dans le présent contexte est à interpréter comme signifiant une teneur en eau libre inférieure à 5 % en poids, de préférence inférieure à 1 % en poids et en particulier inférieure à 0.5 % en poids de la compositions totale.

Dans les agents d'amélioration de la brillance selon l'invention, les quatre résidus d'acides gras d'un tétraester d'acides gras de pentaérythrityle utilisables dans le cadre de l'invention peuvent être identiques ou différents et sont de préférence choisis indépendamment parmi les acides gras saturés ou insaturés, linéaires ou ramifiés, en C₆-C₃₀.

Bien que les résidus d'acides gras d'un tétraester d'acides gras de pentaérythrityle approprié puissent être choisis indépendamment pour donner des tétraesters mixtes, ils sont de préférence identiques.

Comme indiqué précédemment, les résidus d'acide gras des tétraesters peuvent présenter une ou plusieurs insaturations et/ou comporter des ramifications. Ils comprennent d'une manière générale de 6 à 30 atomes de carbone, de préférence de 7 à 25 atomes de carbone et en particulier de 8 à 20 atomes de carbone.

Parmi les tétraesters d'acides gras de pentaérythrityle particulièrement préférés, on peut citer le tétracaprylate, le tétraéthylhexanoate, le tétracaprate, le tétralaurate; le tétracocoate, le tétrastéarate, le tétraisostéarate et le tétraoléate de pentaérythrityle. Comme exemples de tétraesters mixtes, on peut citer notamment l'adipate/caprate/caprylate/heptanoate de pentaérythrityle ou encore l'isostéarate/caprate/caprylate/adipate de pentaérythrityle.

La composition selon l'invention pourra comprendre en outre de 0.1 à 10 % en poids de la composition totale d'une ou de plusieurs matières colorantes ou agents de coloration, comme les pigments et les particules nacrantes (nacres) ou les glitters.

En effet, les inventeurs ont découvert que l'utilisation de ces agents d'amélioration de la brillance dans une formulation de vernis à ongles permettait d'en améliorer la brillance, surtout en réduisant l'effet négatif des composants colorants comme les pigments sur la brillance.

Parmi les pigments utilisables dans l'invention on citera le dioxyde de titane (Cl 77891), l'oxyde de fer noir (Cl 77499), l'oxyde de fer rouge (Cl 77491 ), le DC Red 6 Ba Lake (Cl 15850), le DC Red 7 Ca Lake (Cl 15850:1), le DC Red 34 Ca Lake (Cl 15880), le FDC Yellow 5 Al Lake (Cl 19140), le FDC Blue 1 Al Lake (Cl 42090), le bleu ultramarine (Cl 77007), le DC Violet 2 (Cl 60725), le DC Black 2 (Cl 77266).

Les nacres sont également utilisables dans l'invention, les nacres sont des particules minérales de différentes natures recouvertes d'une ou plusieurs couches d'oxydes (oxydes de titane ou oxydes de fer) ou encore de pigments. Les nacres peuvent se présenter sous la forme d'une poudre ou de plaque de tailles variées. Parmi les nacres utilisables dans l'invention, on peut citer :
- les nacres préparées à partir de mica naturel et ayant subi des opérations de coating successives,
- les nacres préparées à partir de mica synthétique appelé également synthetic fluorphlogopite,
- les nacres préparées à partir de calcium sodium borosilicate,
- les nacres préparées à partir d'oxyde de silice.

Les glitters utilisables dans l'invention peuvent être préparés à partir de plusieurs types de matériaux comme les films polyesters comportant en surface une couche métallique, le polyéthylène téréphtalate, le polybutylène téréphtalate, les copolymères d'acrylates, les copolymères d'acrylates ou encore l'aluminium.

De préférence, ces compositions comprenant en outre un ou plusieurs autres additifs choisis parmi les agents filmogènes, les résines, les plastifiants, les agents rhéologiques, les absorbants UV, les modificateurs de surface, les agents de soins ou agents dits « traitants ».

Les agents filmogènes sont sélectionnés pour leur aptitude à former un film après séchage (évaporation partielle ou totale du ou des solvants). Les différents filmogènes préférés dans le cadre de l'invention sont les suivants :
- Nitrocellulose ayant un taux d'azote compris entre 11.8 et 12.3 % (ce type de nitrocellulose porte généralement le nom de grade RS, il s'agit d'un grade de nitrocellulose soluble dans les solvants de type ester. A l'intérieur de ce grade de type RS, il est possible de trouver des sous-grades qui se différencient notamment par leur poids moléculaire et, par voie de conséquence par la viscosité qu'ils apportent lors de leur mise en solution. Les grades utilisables dans l'invention pourront aller du grade RS 30-35 cps au grade RS 60-80 sec. La nomenclature européenne mentionne des grades de nitrocellulose allant de E24 au grade E130.
- Nitrocellulose ayant un taux d'azote inférieur 11.8 % (ce type de grade porte généralement le nom de grade SS), il s'agit d'un grade de nitrocellulose soluble dans les solvants de type alcool. Le taux d'azote est compris entre 10.7 % et 11.3 %. Comme pour le grade RS, les nitrocelluloses de grade SS peuvent se différencier par leur poids moléculaire et par voie de conséquence par la viscosité de la solution de nitrocellulose dans un solvant approprié.

La nitrocellulose se présente sous la forme d'une poudre blanche humide. La nitrocellulose est en effet commercialisée sous forme « mouillée » (ou « damped ») avec plusieurs types de solvant comme l'éthanol, l'isopropanol ou encore l'eau.

D'autres agents filmogènes utilisables dans l'invention sont :
- L'acétate-butyrate de cellulose. Il existe de nombreux grades de cet agent filmogène qui se différencient par le taux d'estérification de la cellulose mais aussi par le rapport du nombre de fonction ester acétate et du nombre de fonction ester butyrate. Comme pour la nitrocellulose, le cellulose acétate butyrate peut également se différencier par la longueur du polymère cellulose utilisé pour produire le filmogène.
- Ethylcellulose de différents grades de viscosité.
- Hydroxypropylcellulose de différents grades de viscosité.

Les agents filmogènes sont utilisés seuls ou sous la forme de mélange dans la composition dans des proportions allant de 5 % à 20 % en poids de la composition totale.

Les résines permettent de donner du collant et de l'adhérence au film sur l'ongle. Les résines pouvant être utilisées dans l'invention sont les suivantes :
- Résine tosylamide/formaldéhyde (résine toluènesulfonamide/ formaldéhyde) vendue sous la dénomination commerciale Ketjenflex MH, Ketjenflex MS-80 par Akzo Nobel ou encore Sulfonex par Estron.
- Résine tosylamide/époxy (résine toluènesulfonamide/époxy) vendue sous la dénomination commerciale de Lustrabrite S ou Lustrabrite S-70 ou Nagellite 3050 par Telechemische ou encore sous la dénomination polytex resin par Estron.
- Copolymère acide adipique/néopentyl glycol/anhydride trimellitique vendue sous la dénomination commerciale Uniplex 670-P par Unitex ou encore Chempol 509-9514 par Cook Composites
- Copolymère d'anhydride phtalique/glycérine/glycidyl décanoate
- Copolymère d'anhydride phtalique/anhydride trimellitique/glycols vendue sous la dénomination commerciale Polynex Resin par Estron ou encore 7809 Polynex Resin par Degen
- Copolymère de glycérine/acide phtalique
- Copolymère styrène/acrylates/acrylonitrile
- Polymères et copolymères d'acrylates
- Copolymères d'acrylates et de styrène
- Sucrose acétate isobutyrate vendue sous la dénomination commerciale SAIB par Eastman
- Résine polyvinylbutyral.

En plus de leur action collante, la plupart de ces résines ont également une action plastifiante. Les résines sont utilisées dans la formule seules ou sous la forme de mélanges dans des quantités allant de 0.2 à 15 % en poids total de la formulation.

Les plastifiants sont des composés ayant une température d'ébullition élevée qui ne s'évaporent pas lors du séchage du vernis à ongles. Ils sont utilisés pour moduler la dureté du film formé afin d'obtenir les caractéristiques physico-chimiques du film souhaitées et représentent en général de 2 à 9% en poids de la composition totale.

Des exemples de plastifiants utilisables dans l'invention sont l'acétyl tributyl citrate, l'acétyl triéthyl citrate, le tributyl citrate, le triéthylcitrate, le dibutyl phtalate, le triphénylphosphate, la triacétine, le triméthyl pentanyl diisobutyrate, la triéthylhexanoïne, le sucrose benzoate, le dibutyl adipate, le diéthyl phthalate, le diisobutyl adipate, le diisopropyl adipate, le dipropylène glycol dibenzoate.

Parmi les additifs de rhéologie, on citera notamment les dérivés d'argile modifiés comme le stéaralkonium hectorite ou le stéaralkonium bentonite. Ces additifs de rhéologie seront de préférence utilisés de 0.1 à 3 % en poids total de la formulation. Ces additifs permettent notamment la suspension des particules insolubles dans le vernis tels que les pigments ou encore les nacres.

Parmi les absorbants UV, on citera les absorbants de lumière UV qui permettent de protéger la formulation ou les agents colorants utilisés l'invention des rayonnements UV. Parmi les protecteurs UV utilisables dans l'invention, on citera : la benzophénone-1 (CAS 131-56-6), la benzophénone-3 (CAS 131-57-7), le benzyl salicylate (CAS 118-58-1), l'étocrylene (CAS 5232-99-5), le drométrizole (CAS 2440-22-4), le butyl méthoxydibenzoylméthane (CAS 70356-09-1).

Les absorbants UV seront utilisés seuls ou sous la forme de combinaison dont le pourcentage total dans la formule peut aller en général de 0.1 à 1 % en poids.

Parmi les additifs utilisables selon l'invention, on citera également les modificateurs de surface comme les cyclométhicones ou cyclopentasiloxane, les diméthicones et le triméthylsiloxysilicate.

Ces additifs de surface sont généralement utilisés dans des faibles quantités dans la formulation allant de 0.01 à 0.5 % en poids total de la formulation.

On citera également les additifs dits « traitants » de l'ongle utilisable dans la composition selon l'invention. Les additifs traitants peuvent représenter de 0.0001% à 5%, de préférence de 0.01% à 1 % en poids de la composition totale. Parmi ces additifs « traitants », on citera :
- le formaldéhyde utilisé comme durcisseur de l'ongle,
- la vitamine E acétate (tocophéryl acétate),
- la vitamine A palmitate (rétinyl palmitate),
- les dérivés organiques ou inorganiques de calcium tels que le chlorure de calcium, le pantothénate de calcium,
- le diméthyloxobenzodioxasilane vendu sous la dénomination commerciale Pro-DSB par Exsymol,
- la myrrhe,
- les acides aminés soufrés comme la cystéine, ses sels et leurs dérivés, la cystine, le glutathion. Les acides aminés soufrés sont en effet capables de créer des cross-linkings entre la kératine et les groupes SH libres de ces dérivés soufrés,
- la kératine hydrolysée d'origine végétale comme le Prosina de Croda,
- les alpha-hydroxyacides comme l'acide citrique, l'acide ascorbique,
- la biotine,
- l'urée et la diméthylurée.

Dans un aspect supplémentaire, l'invention propose donc l'utilisation comme agents d'amélioration de la brillance d'un ou de plusieurs de ces tétraesters d'acides gras de pentaérythrityle à concurrence de 0.01 à 1 % en poids de la composition totale dans la préparation de compositions cosmétiques anhydres pour vernis à ongles.

L'invention concerne également un procédé de préparation d'une telle composition cosmétique anhydre pour vernis à ongles, dans lequel on mélange au moins un solvant organique cosmétiquement acceptable, une quantité efficace d'au moins un agent d'amélioration de la brillance choisi parmi les tétraesters d'acides gras de pentaérythrityle et de préférence au moins un agent de coloration choisi parmi les pigments, les nacres et/ou les glitters, ainsi qu'éventuellement un ou plusieurs autres additifs choisis parmi les agents filmogènes, les résines, les plastifiants, les agents rhéologiques, les absorbants UV et les modificateurs de surface, les agents dits « traitants ».

Un avantage supplémentaire de l'invention est que les agents d'amélioration de la brillance peuvent non seulement être ajoutés à n'importe quel moment du procédé, c'est-à-dire que l'effet des présents agents n'est pas affecté par l'ordre dans lequel les ingrédients sont dosés. De plus, en complément des avantages déjà mentionnés ci-dessus en relation avec les compositions, il est à remarquer que le procédé ci-dessus peut être réalisé moyennant des installations tout à fait standard et ne demande pas de connaissance, ni de précautions particulières de l'opérateur.

En conclusion, les inventeurs ont découvert d'une manière surprenante que lesdits dérivés de pentaérythritol permettaient de réaliser l'objectif d'augmenter nettement la brillance de compositions cosmétiques anhydres pour vernis à ongles après application.

### Exemples 1 à 8

La préparation d'un vernis à ongles se déroule généralement en deux étapes.

La première consiste à préparer la base, il s'agit en fait d'un vernis à ongles, mais ne contenant pas d'ingrédient colorant. La base se présente sous la forme d'un liquide opaque à laquelle sont ajoutés dans une deuxième étape les pigments, nacres, ainsi que les autres composants susceptibles d'apporter de la couleur à la formulation.

D'une manière générale, il est clairement noté une diminution de la brillance entre la base sans couleur et la composition finale colorée.

La différence de brillance est mesurée par l'utilisation d'un brillancemètre. L'analyse par brillancemètre est réalisée sur des films secs appliqués sur des cartes de contraste normalisées à l'aide d'un applicateur 100 µm (100 µm humide). Le film déposé est séché sous atmosphère et température ambiante pendant une heure. La mesure par brillancemètre 60 ° (le rayon incident fait un angle de 60 ° avec la normale) est réalisée trois fois et la valeur finale obtenue est la moyenne de ces trois mesures.

Les inventeurs ont découvert que l'utilisation de certains dérivés de pentaérythritol (dérivés de 2,2-bis(hydroxyméthyl)-1,3-propanediol) permettait de diminuer la différence de brillance entre la base et la composition finale colorée. L'ajout de ces dérivés de pentaérythritol peut être réalisé à tout moment de la fabrication, aussi bien lors de la première étape de fabrication - la fabrication de la base - que lors de la seconde étape de fabrication de la teinte à savoir le mélange de la base et les ingrédients colorés. Il a toutefois été noté que le meilleur effet et le meilleur résultat de brillance est obtenu lorsque le dérivé de pentaérythritol ou son mélange avec d'autres dérivés sont ajoutés lors de la seconde étape de la fabrication. Le ou les dérivés sont ajoutés lors du mélange de la base avec les divers agents colorants pour atteindre le niveau de brillance maximum.

Il a en outre été noté que les tétraesters de pentaérythritol avaient une plus grande influence sur la brillance de la formulation dans des vernis à ongles contenant des pigments ou contenant des nacres en faible quantité, la brillance de teintes contenant uniquement des nacres étant plus faiblement augmentée.

L'obtention de cette meilleure brillance est rendue possible par l'utilisation d'esters d'acides gras de pentaérythrityle, en particulier ceux listés ci-dessous :
- Le tétracocoate de pentaérythrityle vendu sous la dénomination commerciale DUB PTC par Stéarinerie Dubois.
- Le tétraisostéarate de pentaérythrityle (CAS 62125-22-8) vendu sous la dénomination commerciale Crodamol PTIS par Croda, ou Prisorine 3631 par Uniqema, ou encore Salacos 5418V par Nisshin Oil.
- Le tétraéthylhexanoate de pentaérythrityle (CAS 7299-99-2) vendu sous la dénomination commerciale DUE PTO par Stéarinerie Dubois, ou Salacos 5408 par Nishhion Oil, ou encore NS-408 par Nippon Chemical.
- Le tétraoléate de pentaérythrityle (CAS 19321-40-5) vendu sous la dénomination commerciale DUB PTOL par Stéarinerie Dubois, ou Liponate PO-4 par Lipo.
- Le tétracaprylate/tétracaprate de pentaérythrityle (CAS 68441-68-9) vendu sous la dénomination commerciale Crodamol PTC par Crodamol, ou DUB PTCC par Stéarinerie Dubois, ou Liponate PE-810, ou encore Stepan PTC par Stepan.
- Le tétralaurate de pentaérythrityle (CAS : 13057-50-6) vendu sous la dénomination commerciale DUB PTL par Stéarinerie Dubois ou Pelemol PTL par Phoenix.
- Le tétrastéarate de pentaérythrityle (CAS : 115-83-3) vendu sous la dénomination commerciale DUB PTS par Stéarinerie Dubois ou Liponate PS-4 par la société Lipo.

Ces dérivés sont utilisés à raison de 0.01 % à 1 % en poids, de préférence de 0.05 % à 0.5 % en poids total de la composition. Le pourcentage de tétraester de pentaérythritol à utiliser dans la formulation dépend évidemment entre autres de la quantité totale de pigment et nacre utilisée dans la formulation.

Il a été toutefois noté que le gain de brillance est plus important dans les formulations pour lesquelles les agents de coloration sont majoritairement des pigments. Le gain de brillance est en effet plus faible pour les formulations colorées ne contenant que des nacres.

Ci-dessous dans le Tableau 1, quelques exemples de formulations selon l'invention illustrent l'amélioration de la brillance. La formulation de l'Ex 1 étant donné à titre comparatif et représente une composition connue de l'état de la technique.

La méthode pour déterminer la brillance du film formé est réalisée de la manière suivante :

Le vernis coloré est appliqué sur une carte normalisée à l'aide d'un applicateur permettant la formation d'un film humide d'une épaisseur de 100 mm. Le film est laissé séché pendant 15 minutes à température ambiante. La brillance est mesurée à l'aide d'un brillancemètre sous un angle de 60 ° par rapport à la normale du film. La brillance est notée en % car elle correspond en fait au pourcentage de lumière réfléchie par rapport à la lumière incidente.

**Tableau 1**

| **Ingrédients** | **Ex1** | **Ex2** | **Ex3** | **Ex4** | **Ex5** | **Ex6** | **Ex 7** | **Ex 8** |
|---|---|---|---|---|---|---|---|---|
| Acétate d'éthyle | 25 | 25 | 25 | 25 | 25 | 25 | 25 | 25 |
| Acétate de butyle | 36 | 35.8 | 34.3 | 34.3 | 35.8 | 35.8 | 34.3 | 34.3 |
| Nitrocellulose (70 % Isopropanol) | 17 | 17 | 17 | 17 | 17 | 17 | 17 | 17 |
| Résine polyester | 9 | 9 | 9 | 9 | 9 | 9 | 9 | 9 |
| Copolymère styrène/acrylique | 2 | 2 | 2 | 2 | 2 | 2 | 2 | 2 |
| Acétyl tributyl citrate | 6 | 6 | 6 | 6 | 6 | 6 | 6 | 6 |
| Stéaralkonium hectorite | 1 | 1 | 1 | 1 | 1 | 1 | 1 | 1 |
| Benzophénone1 | 0.5 | 0.5 | 0.5 | 0.5 | 0.5 | 0.5 | 0.5 | 0.5 |
| Diméthicone | 0.5 | 0.5 | 0.5 | 0.5 | 0.5 | 0.5 | 0.5 | 0.5 |
| Ti0₂ | 2 | 2 | 3 | 3 | 1 | | 3 | 3 |
| DC Red 6 Ba Lake | 0.5 | 0.5 | 0.7 | 0.7 | 1 | 1.5 | 0.7 | 0.7 |
| DC Red 7 Ca Lake | 0.5 | 0.5 | 0.7 | 0.7 | 1 | 1.5 | 0.7 | 0.7 |
| pentaérythrityl tétracocoate | | 0.2 | | | | | | |
| pentaérythrityl tétraisostéarate | | | 0.3 | | | | | |
| pentaérythrityl tétraéthylhexanoate | | | | 0.3 | | | | |
| pentaérythrityl tétraoléate | | | | | 0.2 | | | |
| pentaérythrityl tétracaprylate | | | | | | 0.2 | | |
| pentaérythrityl tétralaurate | | | | | | | 0.3 | |
| pentaérythrityl tétrastéarate | | | | | | | | 0.3 |
| Total | 100 | 100 | 100 | 100 | 100 | 100 | 100 | 100 |
| Brillance | 73.2 | 81.5 | 81.9 | 81.3 | 81.6 | 81.9 | 81.6 | 81.3 |

On constate que la brillance est augmentée de façon significative dans les Exemples 2 à 8 par comparaison à l'Exemple 1 non conforme à l'invention.

## Revendications

1. Composition cosmétique anhydre pour vernis à ongles, comprenant au moins un solvant organique cosmétiquement acceptable et une quantité efficace d'au moins un agent d'amélioration de la brillance, **caractérisée en ce que** le au moins un agent d'amélioration de la brillance est un dérivé de pentaérythritol choisi parmi les tétraesters d'acides gras de pentaérythrityle et **en ce que** le au moins un agent d'amélioration de la brillance représente 0.01 à 1 % en poids de la composition totale.

2. Composition selon la revendication 1, dans laquelle les acides gras du tétraester d'acides gras de pentaérythrityle sont identiques ou différents et sont choisis indépendamment parmi les acides gras saturés ou insaturés, linéaires ou ramifiés, en C₆-C₃₀.

3. Composition selon la revendication 2, dans laquelle les acides gras du tétraester d'acides gras de pentaérythrityle sont identiques.

4. Composition selon la revendication 2 ou 3, dans laquelle les acides gras sont choisis parmi le tétracaprylate, le tétraéthylhexanoate, le tétracaprate, le tétralaurate, le tétracocoate, le tétrastéarate, le tétraisostéarate et le tétraoléate de pentaérythrityle.

5. Composition selon l'une quelconque des revendications précédentes, comprenant en outre de 0.1 à 10 % en poids de la composition totale d'un ou de plusieurs agents de coloration choisis parmi les pigments, les nacres et/ou les glitters.

6. Composition selon l'une quelconque des revendications précédentes, comprenant en outre un ou plusieurs autres additifs choisis parmi les agents filmogènes, les résines, les plastifiants, les agents rhéologiques, les absorbants UV, les modificateurs de surface et les agents dits « traitants ».

7. Utilisation d'un ou de plusieurs tétraesters d'acides gras de pentaérythrityle à concurrence de 0.01 à 1 % en poids de la composition totale dans la préparation d'une composition cosmétique anhydre pour vernis à ongles comme agents d'amélioration de la brillance.

8. Procédé de préparation d'une composition cosmétique anhydre pour vernis à ongles selon l'une quelconque des revendications 1 à 6, comprenant le mélange d'au moins un solvant organique cosmétiquement acceptable, d'au moins un agent d'amélioration de la brillance choisi parmi les tétraesters d'acides gras de pentaérythrityle et de préférence d'au moins un agent de coloration choisi parmi les pigments, les nacres et/ou les glitters, ainsi qu'éventuellement d'un ou de plusieurs autres additifs choisis parmi les agents filmogènes, les résines, les plastifiants, les agents rhéologiques, les absorbants UV, les modificateurs de surface et les agents dits « traitants ».

## Claims

1. An anhydrous cosmetic composition for nail varnish, comprising at least one cosmetically acceptable organic solvent and an effective quantity of at least one gloss-enhancing agent, **characterised in that** the at least one gloss-enhancing agent is a pentaerythritol derivative selected from among pentaerythrityl fatty acid tetraesters and **in that** the least one gloss-enhancing agent constitutes 0.01 to 1% by weight of the total composition.

2. A composition according to claim 1, in which the fatty acids of the pentaerythrityl fatty acid tetraester are identical or different and are independently selected from saturated or unsaturated, linear or branched, C₆-C₃₀ fatty acids.

3. A composition according to claim 2, in which the fatty acids of the fatty acid tetraester are identical.

4. A composition according to claim 2 or claim 3, in which the fatty acids are selected from among pentaerythrityl tetracaprylate, tetraethylhexanoate, tetracaprate, tetralaurate, tetracocoate, tetrastearate, tetraisostearate and tetraoleate.

5. A composition according to any one of the preceding claims, furthermore comprising from 0.1 to 10% by weight of the total composition of one or more colouring agents selected from among pigments, pearlescents and/or glitters.

6. A composition according to any one of the preceding claims, furthermore comprising one or more other additives selected from among film-forming agents, resins, plasticisers, rheological agents, UV absorbents, surface modifiers and "treatment" agents.

7. Use of one or more pentaerythrityl fatty acid tetraesters in an amount of 0.01 to 1% by weight of the total composition in the preparation of an anhydrous cosmetic composition for nail varnish as gloss-enhancing agents.

8. A method for preparing an anhydrous cosmetic composition for nail varnish according to any one of claims 1 to 6, comprising mixing at least one cosmetically acceptable organic solvent, at least one gloss-enhancing agent selected from among pentaerythrityl fatty acid tetraesters and preferably at least one colouring agent selected from among pigments, pearlescents and/or glitters, optionally together with one or more other additives selected from among film-forming agents, resins, plasticisers, rheological agents, UV absorbents, surface modifiers and "treatment" agents.

## Patentansprüche

1. Wasserfreie kosmetische Zusammensetzung für Nagellack, die mindestens ein kosmetisch verträgliches organisches Lösungsmittel und eine wirksame Menge mindestens eines Mittels zur Verbesserung des Glanzes umfasst, **dadurch gekennzeichnet, dass** das mindestens eine Mittel zur Verbesserung des Glanzes ein Pentaerythritderivat ist, das aus Pentaerythrityifettsäuretetraestern ausgewählt ist, und dass das mindestens eine Mittel zur Verbesserung des Glanzes 0,01 bis 1 Gew.-% der Gesamtzusammensetzung darstellt.

2. Zusammensetzung nach Anspruch 1, wobei die Fettsäuren der Pentaerythritylfettsäuretetraester gleich oder verschieden sind und unabhängig aus gesättigten oder ungesättigten, linearen oder verzweigten C₆-C₃₀-Fettsäuren ausgewählt sind.

3. Zusammensetzung nach Anspruch 2, wobei die Fettsäuren der Pentarythritylfettsäuretetraester gleich sind.

4. Zusammensetzung nach Anspruch 2 oder 3, wobei die Fettsäuren aus Pentaerythrityltetracaprylat, -tetraethylhexanoat, -tetracaprat, -tetralaurat, - tetracocoat, -tetrastearat, -tetraisostearat und -tetraoleat ausgewählt sind.

5. Zusammensetzung nach einem der vorhergehenden Ansprüche, die außerdem 0,1 bis 10 Gew.-%, bezogen auf die Gesamtzusammensetzung, eines oder mehrerer Farbmittel umfasst, die aus Pigmenten, Perlmutt und/oder Glitzer ausgewählt sind.

6. Zusammensetzung nach einem der vorhergehenden Ansprüche, die außerdem einen oder mehrere weitere Zusatzstoffe umfasst, der bzw. die aus Filmbildnern, Harzen, Weichmachern, rheologischen Mitteln, UV-Absorbern, Oberflächenmodifikatoren und als "Behandlungsmitteln" bezeichneten Mitteln ausgewählt ist bzw. sind.

7. Verwendung eines oder mehrerer Pentaerythritylfettsäuretetraester in bis zu 0,01 bis 1 Gew.-% der Gesamtzusammensetzung bei der Herstellung einer wasserfreien kosmetischen Zusammensetzung für Nagellack als Mittel zur Verbesserung des Glanzes.

8. Verfahren zur Herstellung einer wasserfreien kosmetischen Zusammensetzung für Nagellack nach einem der Ansprüche 1 bis 6, das das Mischen mindestens eines kosmetisch verträglichen organischen Lösungsmittels, mindestens eines Mittels zur Verbesserung des Glanzes, das aus Pentaerythritylfettsäuretetraestern ausgewählt ist, und vorzugsweise mindestens eines Farbmittels, das aus Pigmenten, Perlmutt und/oder Glitzer ausgewählt ist, sowie gegebenenfalls eines oder mehrerer weiterer Zusatzstoffe, der bzw. die aus Filmbildnern, Harzen, Weichmachern, rheologischen Mitteln, UV-Absorbern, Oberflächenmodifikatoren und als "Behandlungsmitteln" bezeichneten Mitteln ausgewählt ist bzw. sind, umfasst.
